# EUROPEAN PATENT APPLICATION

(11) **EP 4 350 353 A1**
(43) Date of publication of application: **10.04.2024**
(21) Application number: 22820171.1
(22) Date of filing: 06.06.2022
(51) Int. Cl.: G01N 33/543, G01N 33/569

(54) **FECAL SAMPLE TEST METHOD AND IMMUNOCHROMATOGRAPHIC TEST PIECE THEREFOR**

(30) Priority: 07.06.2021 JP 2021095332
(71) Applicant: Denka Company Limited, Tokyo 103-8338 (JP)
(72) Inventor: HIRAOKA, Koji, Gosen-shi, Niigata 959-1834 (JP); SAITO, Yuji, Gosen-shi, Niigata 959-1834 (JP)
(74) Representative: J A Kemp LLP
(86) International application number: PCT/JP2022/022712
(87) International publication number: WO 2022/259990

(57) **Abstract**

Disclosed are a method of testing a fecal sample, which method suppresses the effect of interfering substances contained in the fecal sample in immunochromatography, to enable accurate and specific measurement of a substance to be detected in the test sample, and an immunochromatographic test piece therefor. The method of testing a fecal sample includes testing the fecal sample by immunochromatography in the presence of taurodeoxycholic acid or a salt thereof. The immunochromatographic test piece for testing a fecal sample includes taurodeoxycholic acid or a salt thereof in a sample drop region.

## Description

### TECHNICAL FIELD

The present invention relates to a method of testing a fecal sample, and an immunochromatographic test piece therefor.

In recent years, simple test reagents and kits capable of rapidly performing various tests on the presence or absence of infection with pathogens including viruses and bacteria, the pregnancy status, and the like have been developed. Their targets of detection or quantification include pathogen components and human chorionic gonadotropin. Many of the simple test reagents have characteristics that they require no special equipment, that they can be simply handled, and that they are inexpensive. For example, simple test reagents for pregnancy diagnosis are commercially available in general pharmacies. Further, unlike other test reagents, simple test reagents for testing infection with pathogens are widely used in general hospitals and clinics as well as large hospitals and medical testing centers. These facilities are often medical institutions where patients first visit. Therefore, in cases where on-site judgment regarding the presence or absence of infection is possible for samples collected from patients, the patients can be treated in an early stage, so that the simple test reagents are becoming increasingly important in medical care.

Currently, immunoassays using antigen-antibody reaction, especially immunochromatography, are widely known as simple test methods. In immunochromatography, a complex of a capture body (capture substance) that specifically binds to a substance to be detected and a labeling body that specifically binds to the substance to be detected is formed on a membrane, and the label is detected/quantified to detect (measure or quantify) the substance to be detected. Immunochromatography is widely used for detecting a wide variety of substances to be detected, since it can be carried out inexpensively using a simple measurement device.

In one mode of immunochromatography, a sample containing a substance to be detected is dropped onto a test device comprising: a detection section where an antibody that specifically binds to the substance to be detected is immobilized as a capture substance; and a labeling body section containing a labeling body that specifically binds to the substance to be detected; on a strip of a membrane such as nitrocellulose, and the sample is developed while allowing formation of a complex of "substance to be detected - labeling body", followed by capturing the complex in the detection section to detect or quantify the label (for example, Patent Document 1 and

Patent Document 2).

### PRIOR ART DOCUMENTS

### [Patent Documents]

[Patent Document 1] JP 2008-268043 A
[Patent Document 2] JP 2008-203 135 A

### SUMMARY OF THE INVENTION

### PROBLEMS TO BE SOLVED RY THE INVENTION

An object of the present invention is to provide a method of testing a fecal sample, which method suppresses the effect of interfering substances contained in the fecal sample in immunochromatography, to enable accurate and specific measurement of a substance to be detected in the test sample, and an immunochromatographic test piece therefor.

### MEANS FOR SOLVING THE PROBLEMS

As a result of intensive study, the present inventors discovered that, by performing immunochromatography in the presence of taurodeoxycholic acid, the effect of interfering substances can be suppressed to enable accurate and specific measurement of a substance to be detected in a fecal sample regardless of the amount of the fecal sample subjected to the test, thereby completing the present invention.

More specifically, the present invention provides the following.
(1) A method of testing a fecal sample, comprising testing the fecal sample by immunochromatography in the presence of taurodeoxycholic acid or a salt thereof.
(2) The method according to (1), wherein the taurodeoxycholic acid or the salt thereof is contained in a suspension liquid or an extraction liquid for the fecal sample.
(3) The method according to (1), wherein the taurodeoxycholic acid or the salt thereof is contained in a sample drop region of an immunochromatographic test piece used for the immunochromatography.
(4) The method according to any one of (1) to (3), wherein the immunochromatographic test piece used for the immunochromatography contains an anti-norovirus antibody, and detects norovirus in the fecal sample.
(5) An immunochromatographic test piece for testing a fecal sample, the test piece comprising taurodeoxycholic acid or a salt thereof in a sample drop region.

### EFFECT OF THE INVENTION

By the method of the present invention, the effect of interfering substances in a fecal sample can be suppressed to enable accurate and specific measurement of a substance to be detected in the fecal sample regardless of the amount of the fecal sample subjected to the test.

### BRIEF DESCRIPTION OF THE DRAWINGS

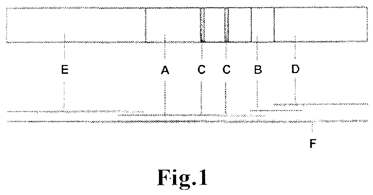

Fig. 1 is a diagram illustrating one preferred mode of a typical immunochromatographic test piece.

### MODE FOR CARRYING OUT THE INVENTION

The present invention relates to a test method which prevents false negatives that occur in fecal samples, and which enables accurate measurement of substances to be detected, and an immunochromatographic test piece therefor. "False negatives" herein means that no signal indicating positivity is produced in an assay regardless of the fact that a substance to be detected is contained in a sample. For example, in immunochromatography, the presence of a substance to be detected in a sample leads to formation of a complex of "immobilized substance - substance to be detected - labeling reagent" on a solid support, and a signal of the labeling reagent is produced from the complex. However, in the cases of false negatives, the production of the signal is inhibited due to reasons such as the absence of formation of the complex.

The immunochromatographic test piece comprises: a support containing a detection zone where an antibody (antibody 1) for capturing a measurement target substance (antigen or the like) is immobilized; a labeling body pad containing a free labeled antibody (antibody 2); a sample pad where a sample is to be dropped; an absorption pad for absorbing a developed sample liquid; and a backing sheet for bonding these members together.

The number of detection zones and the number of types of the labeled antibody contained in the labeling body pad are not limited to one. By using antibodies corresponding to a plurality of measurement target substances, two or more antigens can be measured on the same test piece.

Fig. 1 is a diagram illustrating one preferred mode of a typical immunochromatographic test piece. The immunochromatographic test piece is not limited to the one illustrated in Fig. 1. In Fig. 1, (A) is a support; (B) is a labeling body pad; (C) is a detection zone; (D) is a sample pad; (E) is an absorption pad; and (F) is a backing sheet.

In Fig. 1, a top view is shown in the upper part, and a cross-sectional view is shown in the lower part. In the example shown in the figure, a support in which two detection zones are formed, an absorption pad, a labeling body pad, and a sample pad are layered on a backing sheet. As shown in the figure, one end of the absorption pad and one end of the support are layered on each other; the other end of the support and one end of the labeling body pad are layered on each other; and the other end of the labeling body pad and one end of the sample pad are layered on each other; such that a continuous lateral-flow channel is formed.

The support is a material having a capacity to immobilize an antibody for capturing a test substance (antigen), and also a capacity to allow a liquid to flow in the horizontal direction. The support is preferably a porous, thin film having a capillary action, and is a material capable of transporting, by absorption, a liquid and a component dispersed therein. Examples of the material forming the support include, but are not limited to, cellulose, nitrocellulose, cellulose acetate, polyvinylidene difluoride (PVDF), glass fibers, nylon, and polyketones. Among these, the thin film is more preferably formed using nitrocellulose.

The labeling body pad is composed of a porous substrate containing a labeled antibody. As a material of the substrate, a glass fiber or a non-woven fabric that is commonly used may be used. From the viewpoint of impregnation with a large amount of labeled antibody, the substrate is preferably in the form of a pad having a thickness of about 0.3 mm to 0.6 mm.

The detection zone means a part of the support where the antibody for capturing the test substance (antigen) is immobilized. The detection zone is provided with at least one zone where the antibody for capturing the antigen is immobilized.

The sample pad is a region where the sample or a specimen prepared using the sample is dropped, and is a porous material having water absorbability. As the material, cellulose, glass fibers, a non-woven fabric, or the like may be used. For use in an immunoassay of a large amount of the sample, the sample pad is preferably in the form of a pad having a thickness of about 0.3 mm to 1 mm. It should be noted that the sample pad and the labeling body pad are merely functionally distinguished, and do not necessarily need to be separate materials. Thus, a partial region of the material placed as the sample pad may have the function of the labeling body pad.

The absorption pad is a member for absorbing a component which is supplied to the support but not involved in the reaction in the detection zone. Examples of a material of the absorption pad that may be used include filter papers, sponges, and the like having high water retentivity composed of a common natural polymer compound, synthetic compound, or the like. From the viewpoint of promoting the development of the sample, the material preferably has high water absorbability.

The backing sheet is a member for bonding and fixing all materials described above, that is, the support, the sample pad, the labeling body pad, the absorption pad, and the like such that they partially overlap with each other. The backing sheet is not necessarily required as long as these materials are arranged and fixed at optimal intervals. The backing sheet is, however, preferably used in general from the viewpoint of convenience in the production or use thereof.

In a test piece in the form illustrated in Fig. 1, the sample passes through a porous channel formed by the connection of the series of the sample pad, the labeling body pad, the support, the detection zone, and the absorption pad. Therefore, in this mode, all of these serve as a sample movement region. Depending on the material and the form of each constituent material, there may be a mode in which the sample passes through the interface rather than penetrating the material. Since the sample movement region defined in the present description may be either the inside or interface of the material, a test piece in such a mode is also included within the scope of the present description.

In the present invention, taurodeoxycholic acid or a salt thereof is included in a sample suspension liquid or a sample extraction liquid, or on an immunochromatographic test piece. The salt of taurodeoxycholic acid is preferably a sodium salt or potassium salt. In the following description, "taurodeoxycholic acid or a salt thereof" may be simply referred to as "taurodeoxycholic acid" or "TDOC" unless the context clearly indicates otherwise.

The concentration of taurodeoxycholic acid in the sample suspension buffer or sample extraction buffer is usually 0.005% by mass to 10% by mass, preferably 0.05% by mass to 5% by mass.

The method of applying the taurodeoxycholic acid to the immunochromatographic test piece is preferably a method in which a solution, preferably an aqueous solution using water or an aqueous buffer as a solvent, of taurodeoxycholic acid is added by impregnation or dropping. The amount of the taurodeoxycholic acid added to the immunochromatographic test piece is usually about 0.01 mg to 10 mg, preferably about 0.05 mg to 5 mg.

The fecal sample may be used after dilution with a buffer, or may be used directly without dilution. The substance to be detected is not limited, and may be any substance that is to be detected. Specific examples of the substance to be detected include viral antigens such as those of influenza virus, adenovirus, RS virus, HAV, HBs, HIV, and norovirus; bacterial antigens such as those of MRSA, group A hemolytic streptococcus, group B hemolytic streptococcus, and *Legionella* bacteria; toxins produced by bacteria and the like; mycoplasmas; *Chlamydia trachomatis*; hormones such as human chorionic gonadotropin; C-reactive protein; myoglobin; cardiac troponin; various tumor markers; agricultural chemicals; and antigens such as endocrine disruptors; and also include antibodies against the bacteria, viruses, and the like described above.

The present invention also provides an immunochromatographic test piece formed as described above. The configuration of the test device itself may be a known configuration except that the above-described surfactant is applied to the sample suspension/extraction buffer. Fig. 1 shows a schematic diagram illustrating a specific example of the immunochromatographic test piece of the present invention. However, the test piece of the present invention is not limited thereto.

The following description illustrates a preferred example of an immunoassay using this immunochromatographic test piece. First, a sample is prepared by suspending or extracting a fecal sample in a sample suspension or extraction buffer. The sample is dropped to a sample addition section (D) of a test device comprising: a plastic plate (F); a nitrocellulose membrane (A) layered on the plastic plate (F); a labeling body section (B) on the nitrocellulose membrane (A), the section containing a stabilized dry labeling body pad formed by the above method, in which an antibody capable of antigen-antibody reaction with the substance to be detected is labeled with a colored latex particle; and a detection section (C) in which an antibody as a capture substance capable of antigen-antibody reaction with the substance to be detected is immobilized to form a line. The sample containing the substance to be detected horizontally moves on the membrane while developing the labeling body. Therefore, the presence of the substance to be detected leads to formation of a complex of "substance to be detected - labeling body", and, when the complex reaches the detection section (C), a complex of "capture antibody - substance to be detected - labeling body" is formed on the line. By detecting the presence of the complex based on the colored latex particle in the complex, judgment of the presence or absence of the substance to be detected in the sample is possible. The detection section (C) is a region where an antibody or an antigen-binding fragment thereof is immobilized to form a line, which antibody or antigen-binding fragment is capable of antigen-antibody reaction with the substance to be detected, and in addition, capable of binding to the substance to be detected while allowing the antibody or antigen-binding fragment thereof on the colored latex particle to bind to the substance at the same time. Other components and the like not involved in the reaction are absorbed into the absorption pad section (E). In the example shown in Fig. 1, there are two detection sections (C). These arc, for example, for capturing two kinds of substances to be detected such as type A influenza virus and type B influenza virus. By providing a plurality of such detection sections (C), simultaneous immunoassay of a plurality of kinds of substances to be detected is possible.

Rapid and simple measurement of a substance to be detected is possible by using an immunochromatographic test piece of the present invention that uses, as a stabilized dry labeling body, latex particles to which an antibody or an antigen-binding fragment thereof capable of antigen-antibody reaction with the substance to be detected is bound.

### EXAMPLES

The present invention is described below more concretely by way of Examples and Comparative Examples. However, the present invention is not limited to the following Examples. In the following Examples and Comparative Examples, % is on a mass basis.

### Example 1 Addition to TDOC Sample Suspension Liquid

### 1. Immobilization of Anti-Norovirus Antibody on Nitrocellulose Membrane

A liquid prepared by diluting an anti-norovirus antibody to 1.0 mg/mL with purified water, and an anti-mouse IgG antibody, were provided, and applied to form lines on a nitrocellulose membrane backed with a PET film, such that the anti-norovirus antibody was positioned in the sample pad side, and such that the anti-mouse IgG antibody was positioned in the absorbing body side. The nitrocellulose membrane was then dried at 45°C for 30 minutes to obtain an anti-norovirus-antibody-immobilized membrane. This membrane is referred to as "antibody-immobilized membrane" in the present Example.

### 2. Immobilization of Anti-Norovirus Antibody to Colored Polystyrene Particles

The anti-norovirus antibody was diluted to 0.5 mg/mL with purified water, and colored polystyrene particles were added thereto to 0.1%. After stirring the resulting mixture, carbodiimide was added thereto to 1%, followed by further stirring. After removing the supernatant by centrifugation, the particles were resuspended in 50 mM Tris (pH 9.0), 3.0% BSA, to obtain anti-norovirus-antibody-bound colored polystyrene particles. The particles are referred to as "antibody-immobilized particles" in the present Example.

### 3. Application and Drying of Anti-Norovirus-Antibody-Bound Colored Polystyrene Particles

A predetermined amount, 1.0 µg, of the antibody-immobilized particles prepared in 2 were applied to a glass-fiber non-woven fabric, and the non-woven fabric was then dried at 45°C for 30 minutes. The resulting non-woven fabric is referred to as "dry pad" in the present Example.

### 4. Preparation of Norovirus Test Piece

The antibody-immobilized membrane prepared in 1, and the dry pad prepared in 3 were bonded to other members (a sample pad, a backing sheet, and an absorption pad), and the resulting bonded members were cut into a width of 5 mm, to provide a norovirus test piece. The test piece comprises, from the upstream along the flow of the sample, the sample pad, the dry pad (labeling body pad), the antibody-immobilized membrane (detection zone), and the absorption pad.

### 5. Sample suspension liquid

TDOC (in the Examples and Comparative Examples, sodium taurodeoxycholate) was added to a sample suspension liquid (QuickNavi - sample suspension liquid (for feces), manufactured by Denka Company Limited) to 0.33%, to prepare an improved sample suspension liquid (Example 1). For comparison, a conventional sample suspension liquid, containing no additive, was prepared (Comparative Example 1).

### 6. Treatment of Fecal Sample

A norovirus-positive fecal sample was collected such that the cotton ball of a cotton swab was lightly covered therewith, and the collected sample was suspended in the sample suspension liquid (QuickNavi - sample suspension liquid (for feces), manufactured by Denka Company Limited) (treated fecal sample). Further, a fecal sample was collected such that the cotton ball of a cotton swab was lightly covered therewith, and the collected sample was suspended in the improved sample suspension liquid (improved treated fecal sample).

### 7. Measurement

The norovirus-positive treated fecal sample and improved treated fecal sample were diluted with the sample suspension liquid and the improved sample suspension liquid, respectively, and 50 µL of each resulting dilution was dropped to the test piece. Fifteen minutes later, visual observation was carried out to judge the presence or absence, and the degree, of deposition of colored polystyrene particles on the predetermined position on which the anti-norovirus antibody was immobilized. Cases where the linear deposition could be found were judged as "+", and cases where the deposition could not be found were judged as "-".

**[Table 1]**

| | Dilution factor | | |
|---|---|---|---|
| | x5 | x10 | x20 |
| Comparative Example 1 (treated fecal sample) | + | - | - |
| Example 1 (improved treated fecal sample) | + | + | - |

As shown in Table 1, in Comparative Example 1, 10-fold dilution of the norovirus-positive fecal sample led to failure in the finding of the deposition of the colored polystyrene particles on the predetermined position where the anti-norovirus antibody was immobilized. In contrast, the deposition of the particles could be easily found in Example 1 of the present invention.

### Examples 2 and 3, Comparative Example 2 Test with Fecal Sample

### 1. Immobilization of Anti-Norovirus Antibody on Nitrocellulose Membrane

A liquid prepared by diluting an anti-norovirus antibody to 1.0 mg/mL with purified water, and an anti-mouse IgG antibody, were provided, and applied to form lines on a nitrocellulose membrane backed with a PET film, such that the anti-norovirus antibody was positioned in the sample pad side, and such that the anti-mouse IgG antibody was positioned in the absorbing body side. The nitrocellulose membrane was then dried at 45°C for 30 minutes to obtain an anti-norovirus-antibody-immobilized membrane. This membrane is referred to as "antibody-immobilized membrane" in the present Example.

### 2. Immobilization of Anti-Norovirus Antibody to Colored Polystyrene Particles

The anti-norovirus antibody was diluted to 0.5 mg/mL with purified water, and colored polystyrene particles were added thereto to 0.1%. After stirring the resulting mixture, carbodiimide was added thereto to 1%, followed by further stirring. After removing the supernatant by centrifugation, the particles were resuspended in 50 mM Tris (pH 9.0), 3.0% BSA, to obtain anti-norovirus-antibody-bound colored polystyrene particles. The particles are referred to as "antibody-immobilized particles" in the present Example.

### 3. Application and Drying of Anti-Norovirus-Antibody-Bound Colored Polystyrene Particles

A predetermined amount, 1.0 µg, of the antibody-immobilized particles prepared in 2 were applied to a glass-fiber non-woven fabric, and the non-woven fabric was then dried at 45°C for 30 minutes. The resulting non-woven fabric is referred to as "dry pad" in the present Example.

### 4. Preparation of Impregnated Non-Woven Fabric

TDOC was applied to a non-woven fabric to a predetermined amount, and the non-woven fabric was then dried to obtain an impregnated non-woven fabric (0.25 mg to 0.5 mg of TDOC per test piece) (Examples 2 and 3).

### 5. Preparation of Norovirus Test Piece

The antibody-immobilized membrane prepared in 1, the dry pad prepared in 3, and the impregnated non-woven fabric prepared in 4 were bonded to other members (a backing sheet and an absorption pad), and the resulting bonded members were cut into a width of 5 mm, to provide a norovirus test piece. The test piece using the impregnated non-woven fabric as the sample pad is referred to as "improved test piece" in the present Example. The test piece comprises, from the upstream along the flow of the sample, the sample pad, the dry pad (labeling body pad), the antibody-immobilized membrane (detection region), and the absorption pad.

### 6. Treatment of Fecal Sample

Three norovirus-positive fecal samples were provided ((i) to (iii)). Each norovirus-positive fecal sample was collected such that the cotton ball of a cotton swab was lightly covered therewith, and the collected sample was suspended in a sample suspension liquid (QuickNavi - sample suspension liquid, manufactured by Denka Company Limited) (treated fecal sample).

### 7. Measurement

To a conventional test piece (Comparative Example 2) or an improved test piece (Examples 2 and 3), 50 µL of each norovirus-positive treated fecal sample was dropped. Fifteen minutes later, visual observation was carried out to judge the presence or absence, and the degree, of deposition of colored polystyrene particles on the predetermined position on which the anti-norovirus antibody was immobilized. Cases where a high degree of linear deposition was found were judged as "+"; cases where slight deposition was found were judged as "±"; and cases showing no deposition were judged as "-". The results are shown in Table 2 below.

**[Table 2]**

| Example | Additive | Fecal sample test | | |
|---|---|---|---|---|
| | | (i) | (ii) | (iii) |
| Comparative Example 2 (Conventional test piece) | None | ± | ± | ± |
| Examples 2 and 3 (improved test pieces) | 0.25 mg TDOC (Example 2) | + | + | + |
| | 0.5 mg TDOC (Example 3) | + | + | + |

As shown in Table 2, the conventional test piece of Comparative Example 2 showed only slight deposition of the colored polystyrene particles. In contrast, the improved test pieces of the Examples of the present invention, which used the impregnated non-woven fabric prepared by impregnation with TDOC, allowed easy finding of the particle deposition.

As shown by the above results, the effect of interfering substances in the fecal sample could be suppressed by TDOC, to enable accurate and specific measurement of the substance to be detected in the fecal sample regardless of the amount of the fecal sample subjected to the test.

### DESCRIPTION OF SYMBOLS

(A) Support
(B) Labeling body pad
(C) Detection zone
(D) Sample pad
(E) Absorption pad
(F) Backing sheet

## Claims

1. A method of testing a fecal sample, comprising testing the fecal sample by immunochromatography in the presence of taurodeoxycholic acid or a salt thereof.

2. The method according to claim 1, wherein the taurodeoxycholic acid or the salt thereof is contained in a suspension liquid or an extraction liquid for the fecal sample.

3. The method according to claim 1, wherein the taurodeoxycholic acid or the salt thereof is contained in a sample drop region of an immunochromatographic test piece used for the immunochromatography.

4. The method according to any one of claims 1 to 3, wherein the immunochromatographic test piece used for the immunochromatography contains an anti-norovirus antibody, and detects norovirus in the fecal sample.

5. An immunochromatographic test piece for testing a fecal sample, the test piece comprising taurodeoxycholic acid or a salt thereof in a sample drop region.
